# EUROPEAN PATENT APPLICATION

(11) **EP 4 201 468 A1**
(43) Date of publication of application: **28.06.2023**
(21) Application number: 21217170.6
(22) Date of filing: 22.12.2021
(51) Int. Cl.: A61M 60/17, A61M 60/515, A61M 60/806, A61M 60/237, A61M 60/861, A61M 60/569, A61M 60/531

(54) **A SYSTEM TO TREAT HEART FAILURE WITH PRESERVED EJECTION FRACTION (HFPEF)**

(71) Applicant: Royal College of Surgeons in Ireland, Dublin 2 (IE)
(72) Inventor: Hameed, Aamir, Dublin, 18 (IE); Malone, Andrew, Co. Dublin, A94 D308 (IE); Malone, Grainne, Dublin, 2 (IE)
(74) Representative: Purdylucey Intellectual Property

(57) **Abstract**

A system to treat heart failure with preserved ejection fraction (HFpEF) is described. The system comprises a blood suction device configured for implantation in a left ventricle of a heart of a subject, an anchoring assembly for anchoring the blood suction device to a wall of the left ventricle, and a controller configured to modify the output parameters of the blood suction device so as to activate and deactivate the blood suction device in a pattern synergistic with a cardiac cycle of the subject comprising activation during systole and deactivation during diastole.

## Description

### Field of the Invention

The present invention relates to a system to treat heart failure with preserved ejection fraction (HFpEF). The invention also relates to a method of treating heart failure with preserved ejection fraction (HFpEF), and a method of relieving or preventing secondary pulmonary hypertension.

### Background to the Invention

Heart failure is defined as inability of the heart to supply adequate blood to the body. As the demographics suggest, with increasing ageing population, prevalence of heart failure is also increasing. The cardinal manifestations of HF are dyspnea and fatigue, which may limit exercise tolerance, and fluid retention, which may lead to pulmonary and/ or splanchnic congestion and/or peripheral oedema.

Heart cycle has two phases; a contraction phase when heart pumps the blood to the whole body and the relaxation phase when heart is filled with blood. Heart failure associated with the contraction phase (systole) includes HF with reduced ejection fraction (HFrEF), a condition commonly caused by ischemic heart disease that leads to decrease in stroke volume and cardiac output which results in the activation of neurohormonal response in order to restore the normal cardiac output. Left ventricular assist devices have been developed to assist the heart during the contraction phase, including pump devices designed to assist the pumping of blood into the aorta during diastole (contraction), and contractile devices designed to be implanted between two walls of the left ventricle that reciprocate in a pattern synergistic with the heart rhythm to assist the contraction of the left ventricle during systole.

Heart disease associated with the relaxation phase of the heart cycle (diastole) includes Heart failure with preserved ejection fraction (HFpEF). HFpEF is a clinical syndrome in which patients have symptoms and signs of HF with normal or near normal left ventricular ejection fraction (LVEF >50 percent). During early diastole phase of the cardiac cycle, the healthy LV acts as a vacuum cleaner' that enhances the suction particularly during exercise. In HFpEF, cardiomyocyte stiffness results in the loss of this relaxation enhancement, hence normal LV filling is dependent on high left atrial (LA) pressure to push blood into the LV. This pressure elevation can further cause atrial remodelling and secondary pulmonary hypertension, predisposing patients to develop atrial fibrillation and right ventricular (RV) dysfunction. Each 10 mm Hg increment in pulmonary artery pressure in patients with HFpEF was found to be associated with a 28% increase in 3-year mortality 3. Hence, there is a need to effectively control the death rate from HFpEF.

To date, no pharmacological treatment has yet been shown to reduce morbidity and mortality in patients with HFpEF in randomised clinical trials, mainly due to the pathophysiological heterogeneity of the disease. Current treatment strategies as per the American Heart Association (AHA) and European Society of Cardiology (ESC) focus on treating the comorbidities and use of diuretics to relieve congestion.

Transcatheter left to right interatrial shunt devices (REDUCE LAP-HF-I) have ben proposed as a possible treatment for HFpEF, although right ventricular (RV) volume overloading and subsequent RV failure and worsening of secondary pulmonary hypertension may be an issue with these shunt devices.

A left ventricular implantable device that applies direct internal expansion forces to increase the LV volume (CORolla) has also been proposed as a possible treatment for HFpEF. In theory, expansive elements in the CORolla device could be used for the treatment of all forms of HFpEF, however performance of such a device would likely be heavily dependent on the degree of cardiac remodelling, which varies significantly over HFpEF phenotypes.

It is an objective of the invention to overcome at least one of the above-referenced problems.

### Summary of the Invention

The objective is met by the provision of a system and method for treatment of HFpEF that employs a blood suction device implanted in the left ventricle configured for actuation during diastole to assist the left ventricle pull blood into the left ventricle from the right atrium through the open mitral valve. A controller is operatively coupled to the blood suction device and configured to activate and deactivate the blood pumping device in a pattern synergistic with the cardiac cycle of the subject comprising activation during diastole and deactivation during systole. The controller may receive signals from a sensor, which may form part of the blood suction device or may be a separate sensor such as an ECG. The blood suction device generally comprises an impeller and ideally is an axial flow pump. In use, the blood suction device is anchored to a wall of the left ventricle and positioned such that upon activation the pressure in the top of the left ventricle adjacent the mitral valve is reduced sufficiently to assist the drawing of blood into the left ventricle from the right atrium.

In a first aspect, the invention provides a system comprising:
a blood pumping device comprising configured for implantation in a left ventricle of a heart of a subject;
an anchoring assembly for anchoring the blood pumping device to a wall of the left ventricle; and
a controller configured to modify the output parameters of the blood pumping device so as to activate and deactivate the blood pumping device in a pattern synergistic with a cardiac cycle of the subject comprising activation during diastole and deactivation during systole.

In any embodiment, the system is to treat heart failure with preserved ejection fraction (HFpEF).

In any embodiment, the system is to relieve or preventing secondary pulmonary hypertension.

In any embodiment, the system is to treat a condition associated with impaired filling of the left ventricle during diastole.

In any embodiment, the system comprises at least one sensor in communication with the controller for detecting one or more parameters associated with the heart, wherein the controller is configured to modify the output parameters of the blood pumping device based on the one or more detected parameters.

In any embodiment, the at least one sensor is selected from the group consisting of: a ventricle pressure sensor, a wireless pressure ventricular pressure sensor, a mems pressure sensor, an artery pressure sensor, a cardiac output (CO) sensor, a blood pressure sensor, an ejection fraction of the left ventricle, a heart rate sensor, a motion sensor, an accelerometer, an ECG (Electrocardiogram) sensor, an O2 saturation sensor, a microaccelerometer, and a sonomicrometer..

In any embodiment, the sensor is integral with the blood pumping device.

In any embodiment, the blood pumping device comprises an impeller.

In any embodiment, the blood pumping device comprises an axial flow pump.

In any embodiment, the blood pumping device comprises:
a housing comprising a fluid inlet, a fluid outlet, and a lumen extending through the housing from the fluid inlet to the fluid outlet;
a rotor disposed in the housing;
an impeller disposed on the rotor; and
a motor operably coupled to the rotor for rotation thereof upon activation,
in which the blood pumping device is configured to draw fluid through the housing from the inlet to the outlet upon activation.

In any embodiment, the impeller is disposed on the rotor at a fluid outlet side of the housing.

In any embodiment, the housing is a tubular housing. In any embodiment, the inlet and outlet are aligned along a common axis.

In any embodiment, the motor is an electromagnetic motor comprising a stator disposed within the housing surrounding at least part of the rotor.

In any embodiment, the impeller comprises an axial hub and at least two vanes mounted to the hub, in which each vane has an elongated swept profile.

In any embodiment, each vane has a hub to tip ratio (v) of 0.20 to 0.30.

In any embodiment, the impeller comprises two vanes disposed on opposed sides of the hub.

In any embodiment, each vane has an axial length of 5 to 20 to mm, preferably 10 to 15 mm, and ideally about 12 to about 13 mm.

In any embodiment, each vane has a radial width of 1 to 10 mm, 3 to 7 mm, 4 to 6 mm, or ideally about 5 mm.

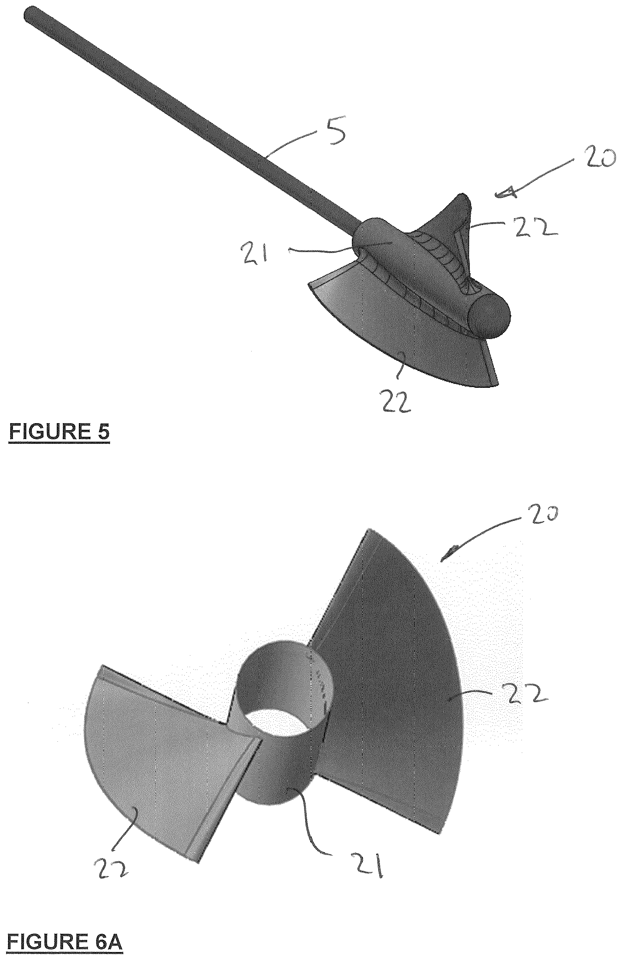

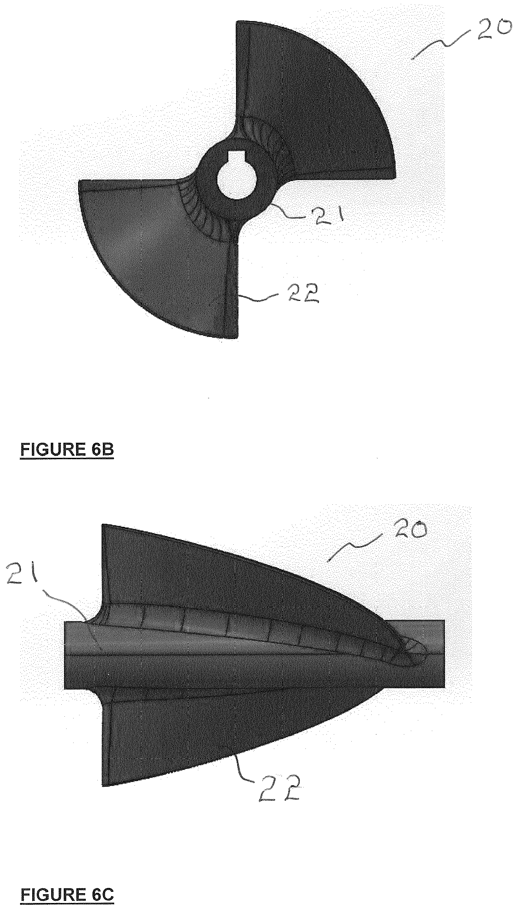

In any embodiment, each vane extends around the hub by a sweep angle of 70° to 140°, 80° to 120°, 90° to 120°, or about 100° to about 120°. The sweep angle refers to the angle that the vane sweeps around the hub. It can be seen in the end view shown in Figure 6B where the vane can be seen to extends around the hub by about 100°.

In any embodiment, a clearance between the radial tip of each vane and the hub housing is 0.1 mm to 1.5 mm, 0.3 mm to 0.7 mm, 0.4 mm to 0.6 mm, and ideally about 0.5 mm.

In any embodiment, the controller is configured to receive heart rate data from a sensor and modify the output parameters of the blood pumping device in real time based on the received heart rate data.

In any embodiment, the controller is configured to compare the heart rate data with reference impeller rotational speed data, calculate an impeller rotational speed based on the comparison, and actuate the blood pumping device during diastole phases to rotate the impeller at the calculated impeller rotational speed.

In any embodiment, the blood pumping device is dimensioned for percutaneous delivery to the left ventricle of the heart inside a delivery catheter of up to 32 Fr.

In any embodiment, the anchoring assembly comprises one or more anchor elements coupled to the blood pumping device.

In any embodiment, the anchoring assembly is configured for mounting to a left ventricular septum, an apex of the left ventricle, or the left ventricular septum and the apex of the left ventricle.

In any embodiment, the system further comprises electronic circuitry for setting the output parameters of the blood pumping device, wherein the electronic circuitry is coupled between the controller and the blood pumping device, and wherein the controller is configured to send control signals to the electronic circuitry to modify the output parameters of the blood pumping device.

In any embodiment, the system further comprises a power unit associated with the blood pumping device.

In any embodiment, the power unit is associated with the electronic circuitry.

In any embodiment, the power unit is an external power unit and wherein the system comprises a power lead to provide power from the power unit to the blood pumping device.

In any embodiment, the controller is configured for implantation within the left ventricle of the heart. In any embodiment, the controller is integral with, or operatively connected to, the blood pumping device.

In another aspect, the invention provides a method comprising the steps of: delivering a blood pumping device to a left ventricle of the heart;
anchoring the blood pumping device to a wall of the left ventricle such that blood pumping device upon activation assists in filling the left ventricle with blood during diastole; and
activating and deactivating the blood pumping device in a pattern synergistic with the cardiac cycle of the subject comprising activation during diastole and deactivation during systole.

In any embodiment, the method is a method of treating heart failure with preserved ejection fraction (HFpEF).

In any embodiment, the method is a method of relieving or preventing secondary pulmonary hypertension.

In any embodiment, the method comprises:
sensing with a sensor a heart rate of the subject; and
activating and deactivating the blood pumping device in a pattern synchronous with the sensed heart rate of the subject.

In any embodiment, the method comprises:
sensing with a sensor a heart rate of the subject; and
activating the impeller of the blood pumping device at an impeller rotational speed appropriate to the sensed heart rate.

In any embodiment, the method comprises:
sensing with a sensor a first heart rate of the subject at a first time point;
activating the impeller of the blood pumping device at a first impeller rotational speed appropriate to the first sensed heart rate;
sensing with a sensor a second heart rate of the subject at a second time point after the first time point; and
activating the impeller of the blood pumping device at a second impeller rotational speed appropriate to the second sensed heart rate,
wherein when the second heart rate is higher than the first heart rate, the second second impeller rotational speed is higher than the first impeller rotational speed.

In any embodiment, the method comprises activating the impeller of the blood pumping device at an impeller rotational speed of 5,000 rpm to 50,000 rpm.

In any embodiment, the method comprises activating the impeller of the blood pumping device at an impeller rotational speed of 10,000 rpm to 40,000 rpm.

In any embodiment, the method comprises reducing the pressure in the left ventricle by at least 5-10 mmHg during diastole.

Other aspects and preferred embodiments of the invention are defined and described in the other claims set out below.

### Brief Description of the Figures

**FIG. 1** is a perspective view of a blood suction device forming part of a system of the invention viewed from a blood inlet side of the device housing.
**FIG.2** is a perspective view of the heart pumping device of Figure 1 viewed from a blood outlet side of the device housing.
**FIG. 3** is a sectional view of the blood suction device of Figure 1.
**FIG. 4** is a sectional illustration of a blood suction device of Figure 1.
**FIG. 5** is a perspective view of a rotor forming part of the blood suction device of Figure 1.
**FIG. 6A** is a perspective view of a swept vane impeller forming part of the rotor of FIG. 5.
**FIG. 6B** is a end elevational view of a swept vane impeller forming part of the rotor of FIG. 5.
**FIG. 6C** is a side elevational view of a swept vane impeller forming part of the rotor of FIG. 5.
**FIG. 7** is an illustration of a human heart with a system of the invention implanted in the left ventricle and anchored to apex of the left ventricle.
**FIG. 8** is an exploded view of the left ventricle of FIG. 7 showing the blood suction device in-situ in the left ventricle with the inlet facing the mitral valve and showing how the device pulls blood into the LV from the RA when activated during diastole.
**FIG. 9** is an illustration of one embodiment of the system of the invention in which the controller is located externally of the heart and is electrically connected to the blood suction device by a control wire and electrically connected to an ECG sensor placed on the subject's chest.
**FIG. 10** is an illustration of another embodiment of the system of the invention in which the controller is located externally of the heart and is electrically connected to the blood suction device by a control wire and electrically connected to a heart rate sensor implanted on an external wall of the left ventricle.
**FIG. 11** is an illustration of another embodiment of the system of the invention in which the controller is located externally of the heart and is electrically connected to the blood suction device by a wireless transmitter/receiver and in which the sensor is coupled to the blood suction device and in contact with an internal wall of the left ventricle.
**FIG. 12** shows a block diagram of one embodiment of the main components of the system of the invention deployed for use in the heart.
**FIG. 13** shows a block diagram of another embodiment of the main components of the system of the invention deployed for use in the heart.
**FIG. 14****:** A graph of impeller tip diameter vs rotor speed. The speed at which the minimum impeller diameter falls within our maximum diameter range is indicated at 38348 rpm.
**FIG. 15****:** A semi log plot of pump flow parameters with respect to rotor speed. The minimum required rotor speed based on our maximum impeller diameter is indicated at 38348 rpm.
**FIG. 16****:** A graph of the pump coefficients with respect to specific speed. The minimum specific speed based on our maximum diameter is indicated at 1398.5.
**FIG. 17****:** A graph of NPSHR and Head vs specific speed for an assumed impeller tip speed (u = 13.272 m min⁻¹).

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

Additionally, the terms "treatment" or "treating" refers to an intervention (e.g. the use of the device of the invention to assist systole of the left ventricle) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Blood suction device" means a device configured to implantation in a chamber of the heart, particularly the left ventricle, that upon activation can draw blood into the left ventricle from the right atrium through the mitral valve during diastole. The device generally comprises an impeller. The impeller comprises a rotor with vanes disposed within a housing with a blood inlet and a blood outlet. The housing is generally tubular and elongated wherein the blood inlet is disposed at one end of the housing and the blood outlet is disposed at an opposite end of the housing. The impeller is mounted for rotation within the housing with the vanes disposed towards the blood outlet of the housing. In use, the device is anchored to a wall of the left ventricle such that the blood inlet of the housing is positioned closer to the mitral valve than the blood outlet of the housing. The housing typically has a longitudinal axis that is directed towards the mitral valve. The device includes a motor, which may be an electromagnetic motor comprising a stator. The device may also include the controller and a sensor. The device may include or be operatively coupled to an inductive charging apparatus.

"Output parameters" as applied to the blood suction device means frequency of activation, impeller rotational speed during activation. It will be appreciated that during phases of deactivation of the blood suction device that the impeller rotation may be maintained but at a rotational speed that is significant reduced compared with an activation phase. Thus, for example the impeller may rotate at a rotational speed of 50% or less than the activation phase rotational speed.

"Anchor" means an element configured for engaging tissue, for example a wall, septum or apex of the left ventricle of the heart. The anchoring assembly may comprise one, two, three or more anchors. One may attach to the left ventricular septum and one may attach to the apex of the left ventricle. The anchoring assembly comprises anchors that are configured to be deployed and embedded in the tissue of the wall of the left ventricle to ensure a secure attachment of the blood suction device. Anchoring is usually at a minimum of two points in the wall. The anchors are adapted to withstand repeated loads generated by both the heart suction device, as well as loads generated by the tissue in which they are embedded. In addition, the anchors are designed to minimise damage to the surrounding tissue, through designs that minimise the damage caused through penetration of the anchor through muscle tissue, and any subsequent damage to the tissue generated by repeated and continuous contraction cycles. The anchor may be formed from any suitable structure. Anchors are selected according to tissue type and the condition of the tissue. In one embodiment, the anchor is encapsulated with an elastic polymer membrane that expands circumferentially when the anchoring assembly is compressed at deployment configuration.

In one embodiment, the coupling between at least one of the anchors and the blood suction device comprises a magnet or magnetisable element. This facilitates coupling of the anchor and the blood suction device at a target location in-vivo. In one embodiment, the anchors are configured for detachable coupling with the heart suction device. This allows one or both anchors to be delivered to the target location separately from the heart suction device. In one embodiment, the device is delivered in two parts, a first part comprising a first anchor, and a second part comprising the heart suction device coupled to the second anchor.

The system of the invention may include an inductive charging apparatus. "Inductive charging apparatus" means an apparatus for charging the system, or any device of the system, wirelessly by using any one of electromagnetic field, wireless radio waves or magnetic resonance charging to transfer energy to charge the device. For example, the blood suction device may comprise an inductive charging apparatus.

The system of the invention is designed to deliver clinically effective functional support to the left ventricle of the heart during the filling phase (diastole). The system increases the flow of blood into the left ventricle of the heart during diastole in which it is implanted and then is deactivated during systole. The controller is programmable such that the system can act as an auto-adaptive system that responds to physiologic cues. The system can continuously adapt to specific user requirements. The blood suction device is configured by means of the controller to activate in a pattern synergistic to the natural cycle of the heart to, for example, activate the blood suction device during diastole and deactivate the blood suction device during systole. Thus, as the heart rate of a subject increases, the frequency of activation and deactivation of the blood suction device changes in sync with the heart rate based on signals received from the sensor. In addition, the controller can modify the impeller rotation speed based on physiological cues received from the sensor, for example increase the impeller rotational speed during periods of elevated heart beat and reduce the impeller rotational speed during periods of reduced heart rate. The controller may comprise a computation device configured to receive data from the sensor, compare the stored with stored reference data, and modify the output parameters of the blood suction device based on the comparison.

The controller may be configured for use outside the heart. Thus, the system may include a control lead that operatively couples the heart suction device implanted within the heart with the controller located outside the body. The control lead may extend through a wall of the heart and through the chest to the external controller. In another embodiment, the control lead may extend percutaneously from the left ventricle through part of the vasculature and out of the body at a suitable location. The controller may be configured to be wearable by a subject. The controller may be connected to a power source, for example a battery. In another embodiment, the controller may be configured for implantation within the heart, as part of the blood suction device, and be configured to receive data from the sensor.

The sensor may be part of the system of the invention, or the system of the invention may be configured for use with a separate sensor. For example, the system may include a sensor configured for implantation in the left ventricle of the heart where it is operatively connected or coupled to the blood suction device. This may be a wire/sensor element configured to extend into contact with a wall of the heart for sensing a parameter of heart contraction/relaxation or configured to contact blood in the left ventricle or another chamber to detect a parameter of the blood (for example pressure or flow rate).

In another embodiment, the sensor is configured for implantation on an external wall of the heart and operatively coupled to the controller, which may be mounted externally of the subject's body.

In any embodiment, the system comprises a graphical user interface which may form part of the controller. The controller may be configured to display on the GUI data relating to the functioning of the system, for example heart rate, blood pressure, frequency of activation of the impeller, rotational speed of the impeller.

In any embodiment, the sensor is configured to detect ejection fraction of the left ventricle. In any embodiment, the controller is configured to compare the detected ejection fraction of the left ventricle with one or more ejection fraction reference values and modify the output parameters of the blood suction device based on the comparison. In any embodiment, the controller is configured to switch off the blood suction device for a period of time when the ejection fraction is detected to be reduced. In any embodiment, the controller is configured to resume operation of the blood suction device when the ejection fraction is detected to be preserved.

The system may further include telemetric components for transmitting and receiving signals relating to the activity of the left ventricle, heart or the activity of the system. Telemetric components may include wireless medical telemetric elements using radio frequency to relay data such as pulse, heart rate, and electrical activity of the heart to the controller.

The controller may be configured to control the operation of the blood suction device either through automatic execution of program instructions in memory and/or upon receiving an external input from a user. The memory component of the system may include ROM and RAM memory. The controller may take the form of a microprocessor.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

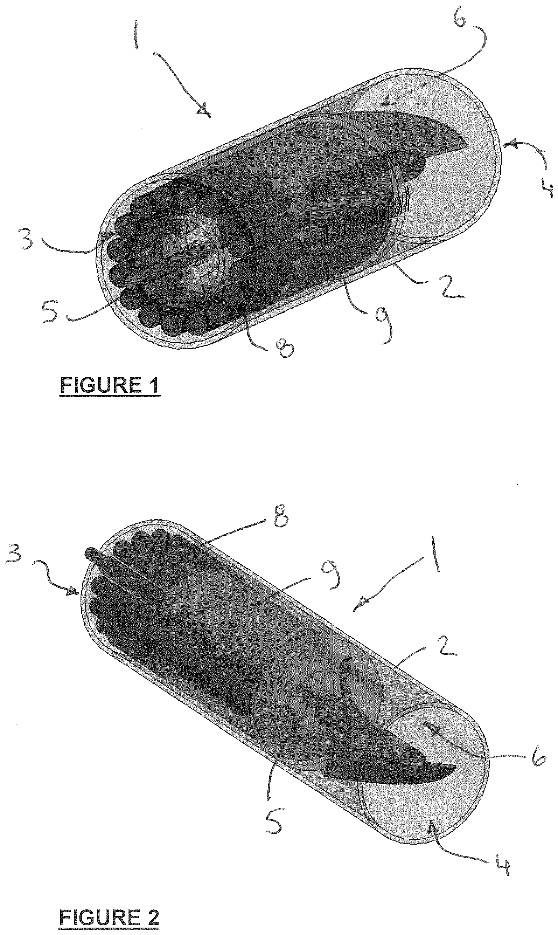

Referring to the drawings and initially to Figures 1 to 6, there is illustrated a blood suction device forming part of a system of the invention, and indicated generally the reference numeral 1. The device 1 comprises an external tubular housing 2 with a blood inlet 3, blood outlet 4, and internal lumen 6 extending between the inlet 3 and outlet 4. The housing has a length of 30 mm and a diameter of 7.6 mm. A rotor 5 is mounted on spaced apart bearings 7 and extends axially through the internal lumen 6 for rotation within the lumen. A stator 8 is mounted within the housing adjacent the blood inlet 3 surrounding a proximal end of the rotor and in use functions to rotate the rotor during activation of the blood pumping device. A PCB 9 comprising electronic circuitry for the operation of the device is disposed in a central part of the housing surrounding the rotor 5.

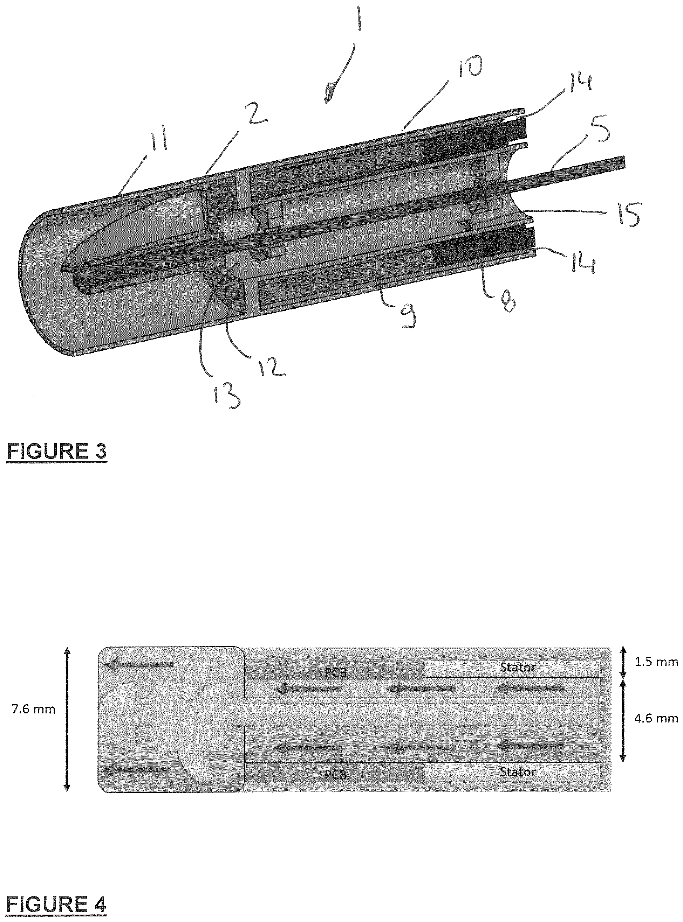

Referring to Figure 3, the housing 2 has a proximal part 10 and distal part 11 separated by a dividing wall 12 with a central annulus 13. The proximal part of the housing comprises an annular compartment 14 that contains the stator 8 and PCB 9 and a central lumen 15 providing a passageway for blood during activation of the device. The PCB 9 includes the following components: resistor, diode, transistor, microprocessor, pressure sensors.

Referring specifically to Figures 4 to 6, an impeller device 20 is mounted to a distal end of the rotor 5 and comprises a tubular hub 21 with a diameter of 1.665 mm and two elongated swept vanes 22 attached to opposite sides of the hub in a symmetrical manner. Each vane has an axial length of 13 mm and a radial width of 4.995 mm and sweeps around the hub along its length from a distal end to a proximal end at an angle of about 100° (sweep angle) illustrated as Ø in Figure 6B. The clearance between each vane and the surrounding external housing is 0.5 mm.

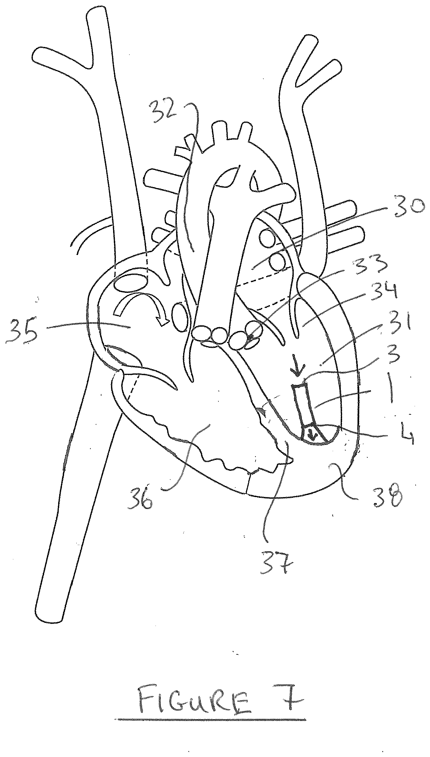

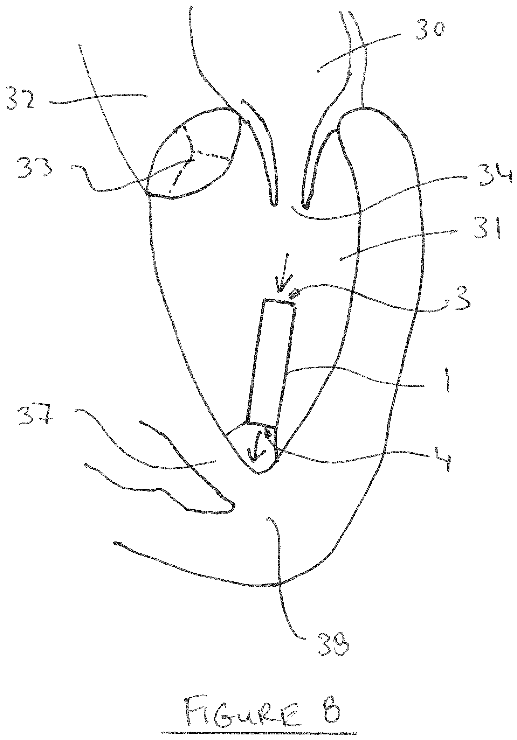

Figures 7 and 8 illustrate a human heart including left atrium 30, left ventricle 31, aorta 32 and aortic cusps 33, mitral valve 34, right atrium 35, right ventricle 36, left ventricular septum 37 and left ventricular apex 38. The blood suction device 1 is shown implanted in the left ventricle with two anchoring arms, a first anchoring arm 39A anchored in the lower part of the left ventricular septum 37 and a second anchoring arm 39B anchored to the apex 38. The device is positioned such that the blood inlet 3 of the device 1 faces towards the mitral valve 34 and left atrium and the blood outlet 4 faces the apex of the left ventricle. Upon activation of the device during diastole, blood is drawn into the device through the inlet 3 and ejected through the outlet 4 as illustrated by the arrows A, which assists the emptying of blood from the right atrium into the left ventricle. Once diastole is completed, the device is deactivated as the mitral valve closes and the aortic cusps open, and the left ventricle contracts to pump blood into the aorta unassisted.

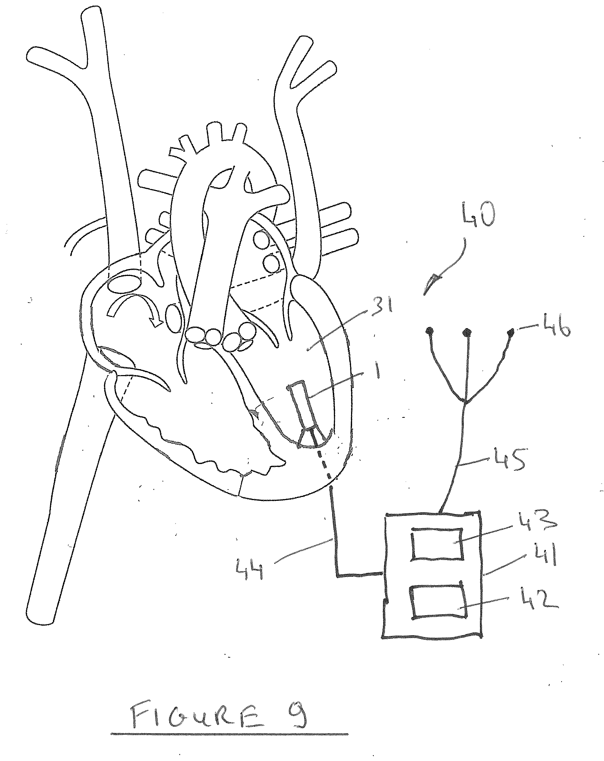

Figures 9 to 11 illustrate some embodiments of the system of the invention, in which parts described with reference to the previous embodiments are assigned the same reference numerals.

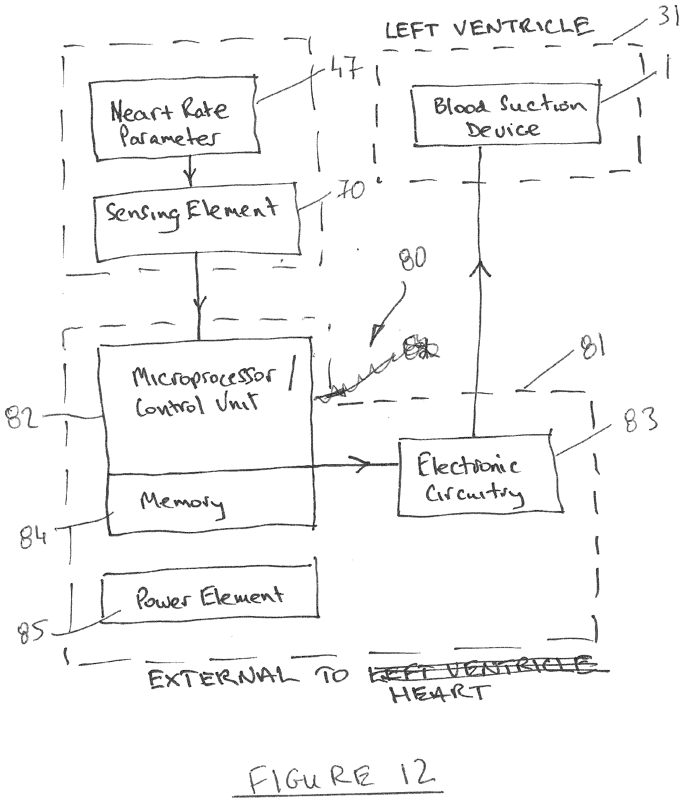

In Figure 9, a system of the invention 40 comprises a blood suction device 1 shown implanted in the LV of the heart as described previously and a controller device 41 external to the subject's body containing a power module 42 and a controller/processing device 43. A control lead 44 extends from the LV through a wall of the LV and operatively connects with the controller device 41. A sensing lead 45 operatively connects the controller device with an ECG sensor 46 mounted to the subject's chest. The ECG array has one or more leads that are placed on the skin of the user, as shown in Figure 12. The ECG leads may be positioned subcutaneously or implanted, such as for example in the left parasternal region of the body. In this embodiment, the ECG monitors the electrical parameters of the subject heart, the controller receives the electrical parameters and activates and deactivates the blood pumping device 1 in a pattern synergistic with the electrical parameters to activate the blood suction device during diastole and deactivate the blood suction device during systole to assist the LV ventricle fill with blood during diastole. The controller also controls other parameters of the blood suction device based on data received from the sensor, including increasing the impeller rotational speed during diastole during periods of activity (e.g., when the heart-beat rises above a resting heart rate) and lowering the impeller rotational speed during diastole during periods of rest.

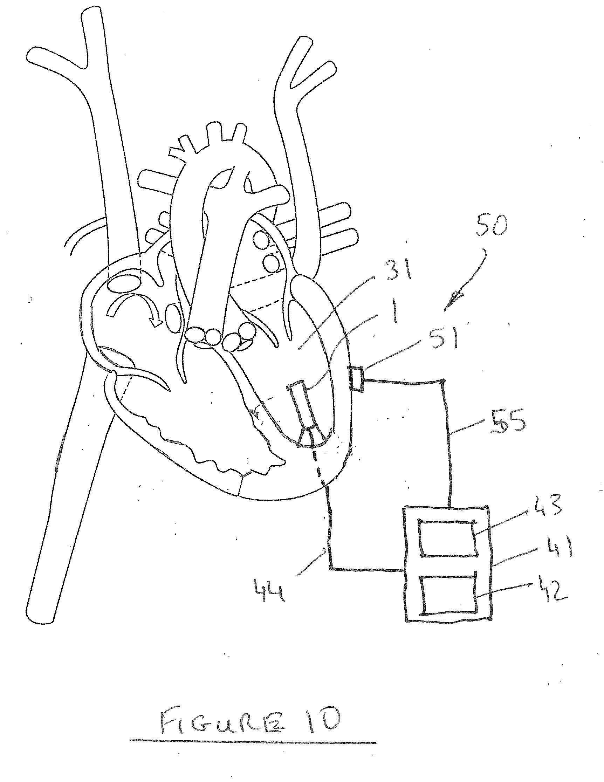

Figure 10 illustrates an alternative embodiment of the system of the invention, indicated by the reference numeral 50 in which parts described with reference to the previous embodiments are assigned the same reference numerals. In this embodiment, a sensor 51 to detect left ventricle contraction parameters is implanted in the subject's chest by means of keyhole surgery and anchored using a suitable anchoring means on an external wall of the left ventricle. A sensor lead 55 extends between the sensor 51 and the controller device 41 to provide data comprising left ventricle contraction parameters to the controller. The use of the system to augment the filling of the left ventricle during diastole is the same as that described previously with reference to Figure 9.

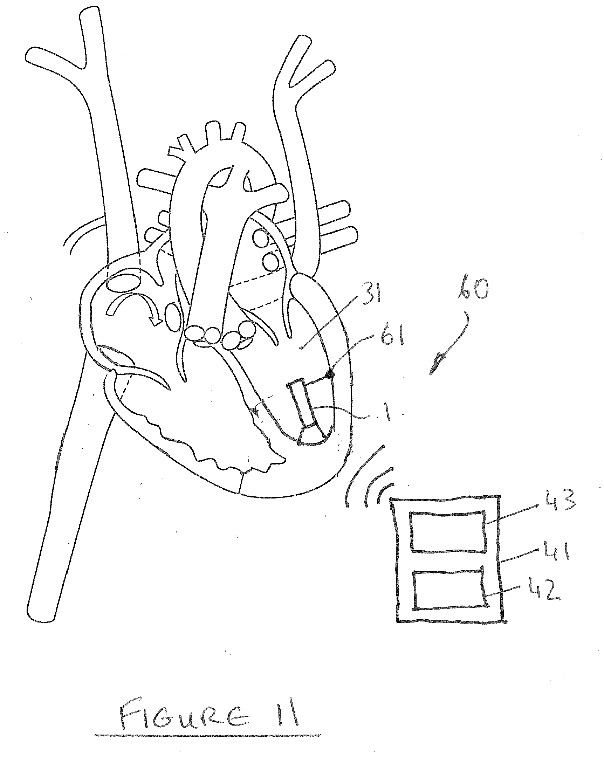

Figure 11 illustrates an alternative embodiment of the system of the invention, indicated by the reference numeral 60 in which parts described with reference to the previous embodiments are assigned the same reference numerals. In this embodiment, a sensor 61 is provided in the form of a wire connected to the housing of the blood suction device 1 configured to deploy upon deployment of the device in the left ventricle into contact with a wall of the left ventricle. The wire is configured to detect electrical parameters of the heart and relay the detected electrical parameters to a wireless transmitter/receiver mounted in the housing of the blood suction device. The controller includes a wireless receiver/transmitter to receive the data from the sensor 61 and to relate output parameters to the blood suction device. The use of the system to augment the filling of the left ventricle during diastole is the same as that described previously with reference to Figure 9.

Figure 12 shows a block diagram of one embodiment of the main components of the system of the invention. In the embodiment shown in this figure, the blood suction device 1 comprising an impeller pump is anchored in the left ventricle 31 of the subject's heart. A sensor 70 configured to detect a heart rate parameter is located outside of the heat. The sensor may be an ECG sensor comprising ECG leads disposed on the subject's chest, or another type of heart rate parameter sensor located externally of the subject's heart. The sensor 70 is configured to detect a heart rate parameter 47, in this case left ventricle contraction parameters.

The sensor data output from the sensor 70 is communicated to the microprocessor/control unit 81 by means of either wired or wireless communications. The microprocessor/control unit 81 is configured to continuously assess the time of diastole and systole based on the received sensor data and optimally synchronise the activation and deactivation of the blood suction device 1 into sync with the diastole and systole stages of the cardiac cycle.

In use, the sensor 70 detects the heart rate parameters of the heart and sends the sensor data to the microprocessor/control unit 82. The received sensor data is analysed by the microprocessor/control unit 82 according to the instructions stored in the memory 84. The microprocessor/control unit 82 then delivers control signals to the power unit 85 and to the electronic circuitry 83 so as to modify the output parameters of the heart suction device 1 as appropriate based on the sensor data so as to activate the heart suction device 1 in a pattern synergistic to the natural contraction cycle of the heart to activate the device during diastole and deactivate the device during systole.

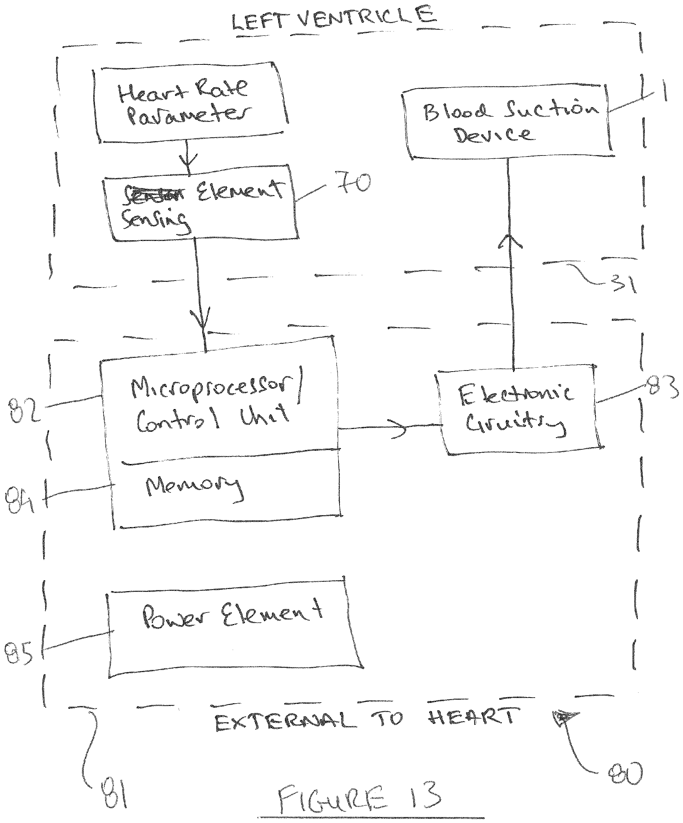

Figure 13 shows a block diagram of another embodiment of the main components of the system of the invention. In this embodiment, which is substantially the same as the system described with reference to Figure 11, the sensor 70 for sensing a heart rate parameter is located within the left ventricle of the heart and sends sensed data to the external microprocessor/control unit 81 by means of wired or wireless communication. The use of this embodiment of the system is substantially the same as that described previously with reference to Figure 11.

### Delivery of the Blood Suction Device to the LV

The device may be delivered percutaneously via femoral artery/subclavian artery or femoral vein/subclavian vein. The device may also be delivered through transapical route, whereby an incision is given in an intercostal space and the device is delivered through the apex.

### Supporting Data

Figure 4 illustrates a blood suction device according to one embodiment of the invention. The embodiment of Figure 4 is designed to be suitable for delivery in a 29 Fr (9.66 mm OD) catheter to give more space for internal components such as a printed circuit board and stator coils. The wall thickness is customisable to a certain extent, but we will assume a wall thickness of 1 mm. The internal diameter is therefore 7.66 mm. Assume 0.5 mm for clearance and wall thickness, our impeller tip diameter cannot exceed 6.6 mm.

The pump parameters are based on a series of linked equations found in Gülich (2020 - Page 455) [Gülich, J., 2020. Centrifugal Pumps. 4th ed. Springer International Publishing, p.455.]

The quantities already known are Flow rate (Qopt), Head (Hopt), Net Positive Suction Head Available (NPSHA), Cavitation Safety Factor (FNPSH), and Pump Efficiency (ηh). The values and any calculations described are included in the MATLAB script Pump_graph.m.

The flow rate required by the pump is equal to the volume of blood the pump needs to move in one heartbeat multiplied by the heart rate, expressed in m3 s-1. Assuming 20ml of blood must be moved and a worst-case heart rate of 200 bpm; Qopt = 6.66x10-5 m3 s-1.

The required head is the pressure reduction necessary to alleviate LA hypertension in HFpEF. This is estimated to be 10 mm Hg in the worst-case scenario. This pressure can be expressed in metres by dividing by the specific weight of the fluid (assumed to be the same as water); Hopt = 0.1359 m.

The Net Positive Suction Head Available is equal to the net pressure at the inlet of the pump minus the vapour pressure of the fluid expressed in metres. The total pressure in the LA is taken as the sum of the LAP at diastole (20 mm Hg - this is when the pump will be active), the height of the LA (3 cm), and atmospheric pressure (760 mm Hg). The vapour pressure of blood is assumed equal to that of water (47 mm Hg). NPSHA = 9.995 m.

The cavitation safety factor is estimated to limit the amount of cavitation by ensuring the pressure does not drop below the blood vapour pressure, this is estimated as FNPSH = 0.8*NPSHA.

The pump efficiency cannot be calculated and must be derived experimentally. For axial flow pumps typically ηh = 85%.

The desired quantities here are the rotor speed (n), the impeller tip diameter (d2), the hub ratio (v), the specific speed (nq), the pressure coefficient (ψ), the flow coefficient (ϕ), and the cavitation coefficient (σ).

The quantities can be calculated iteratively, but in practice it is simpler to use a MATLAB script to plot the quantities with increasing rotor speed and determine the possible pump characteristics based on the generated graphs of these parameters.

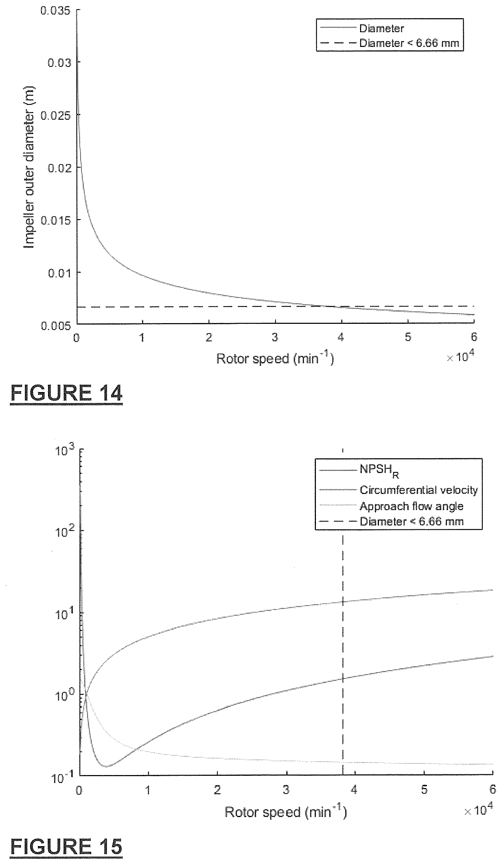

Figure 15 shows the calculated minimum impeller diameter for a range of rotor speeds. By selecting a value of diameter less than or equal to our maximum impeller diameter (6.66 mm) we can determine the minimum rotor speed required for the pump to be 38348 rpm.

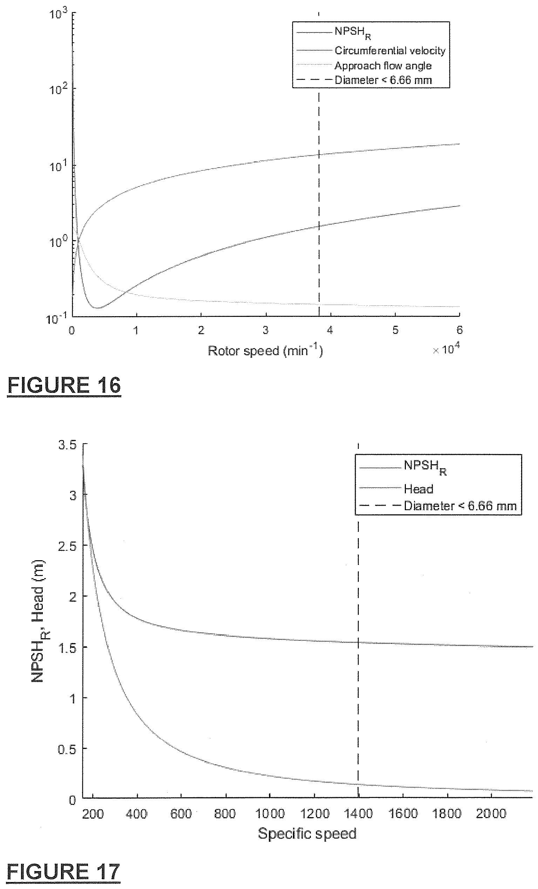

Figure 16 is a semi log graph of the flow parameters with respect to rotor speed.

Again, the minimum required rotor speed is indicated at 38348 rpm. This graph indicates that the parameters at the required rotor speed are: NPSHR = 1.5322 m, circumferential velocity u = 13.272 m min-1, and approach flow angle β = 0.1455°.

Figure 17 shows the pump coefficients with respect to specific speed. Here the minimum required rotor speed has been converted to specific speed and indicated on the graph at 1398.5. The values of the coefficients can be evaluated at this point on the graph as: Ψ = 0.0151, ϕ = 0.1465, v = 0.0608, and σ = 0.1708.

Figure 18 shows NPSHR and pump head vs specific speed when the impeller tip speed is set to the calculated circumferential velocity evaluated previously (u = 13.272 m min-1). The required specific speed is indicated at 1398.5 and evaluating NPSHR and head at this value yields 1.5332 m and 0.1360 m respectively. This value for NSPHR is the same as evaluated from Figure 4 and the value for head is the same as specified as a requirement for the pump.

Figure 7 shows a table taken from Gülich (2020) pg. 456 which outlines the number of impeller blades and diffuser vanes required for ranges of specific speeds. It can be seen from the table that because our specific speed > 290, the number of impeller blades should be 2 and the number of diffuser vanes should be 5/7/9.

Rather than work through the calculations manually for the blade profiles, a software tool called CFTurbo (CFturbo GmbH, Germany) was used to finalise blade design. This tool takes in information calculated in the previous section and semi-automates the process of impeller design. The inputs selected were as calculated for a 200 bpm HFpEF condition as stated previously. However, the hub ratio calculated for this condition was too small to be practical. Therefore, the code was run again for a range of different heart rates and a compromise hub ratio of 0.25 was selected. The final impeller design was exported and prepared for inclusion in the full CAD file.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A system (40, 50, 60) to treat heart failure with preserved ejection fraction (HFpEF), the system comprising:
a blood suction device (1) configured for implantation in a left ventricle (31) of a heart of a subject, in which the blood suction device is configured to draw blood from a left atrium (30) into the left ventricle (31) of the heart through a mitral valve (34) upon activation;
an anchoring assembly (39A, 39B) for anchoring the blood suction device to a wall of the left ventricle; and
a controller (41, 82) configured to modify the output parameters of the blood suction device so as to activate and deactivate the blood suction device in a pattern synergistic with a cardiac cycle of the subject comprising activation during diastole and deactivation during systole.

2. A system according to Claim 1 or 2, further comprising at least one sensor (46, 51, 61, 70) in communication with the controller for detecting one or more parameters associated with the heart, wherein the controller is configured to modify the output parameters of the blood suction device (1) based on the one or more detected parameters.

3. A system according to Claim 2, in which the at least one sensor is selected from the group consisting of: a ventricle pressure sensor, a wireless pressure ventricular pressure sensor, a mems pressure sensor, an artery pressure sensor, a cardiac output (CO) sensor, a blood pressure sensor, a heart rate sensor, a motion sensor, an accelerometer, an ECG (Electrocardiogram) sensor, an O2 saturation sensor, a microaccelerometer, and a sonomicrometer..

4. A system according to Claim 2 or 3, in which the sensor is integral with the blood pumping device and configured for implantation within the left ventricle of the heart.

5. A system according to any preceding Claim, in which the blood suction device comprises an impeller (20).

6. A system according to any preceding Claim, in which the blood suction device comprises an axial flow impeller (20).

7. A system according to any preceding Claim, in which the blood suction device (1) comprises:
a housing (2) comprising a fluid inlet (3), a fluid outlet (4), and a lumen (6) extending through the housing from the fluid inlet to the fluid outlet;
a rotor (5) disposed in the housing;
an impeller (20) disposed on the rotor at a fluid outlet end of the housing; and
a motor operably coupled to the rotor for rotation thereof upon activation,
in which the blood suction device is configured to draw fluid through the housing from the inlet to the outlet upon activation.

8. A system according to Claim 7, in which the motor is an electromagnetic motor comprising a stator (8) disposed within the housing surrounding at least part of the rotor (5).

9. A system according to Claim 7 or 8, in which the impeller (20) comprises an axial hub (21) and at least two vanes (22) mounted to the hub, in which each vane has an elongated swept profile.

10. A system according to any of Claims 7 to 9, in which each vane (22) comprises a hub to tip ratio (v) of 0.20 to 0.30.

11. A system according to Claim 9 or 10, in which the impeller (20) comprises two vanes (22) disposed on opposed sides of the hub.

12. A system according to any of Claims 9 to 11, in which each vane (22) has an axial length of 10 to 15 mm and a radial width of 3 to 7 mm.

13. A system according to Claim 10, in which each vane (22) extends around the hub along a sweep angle of 80° to 120°.

14. A system according to any preceding Claim, in which the controller (41, 82) is configured to receive heart rate data from a sensor and modify the output parameters of the blood suction device in real time based on the received heart rate data.

15. A system according to Claim 14, in which the controller is configured to compare the heart rate data with reference impeller rotational speed data, calculate an impeller rotational speed based on the comparison, and actuate the blood suction device during diastole phases to rotate the impeller at the calculated impeller rotational speed.
